(19) Europäisches Patentamt / European Patent Office / Office européen des brevets

(11) **EP 3 543 359 B1**

(12) **EUROPEAN PATENT SPECIFICATION**

(45) Date of publication and mention of the grant of the patent:
**14.02.2024 Bulletin 2024/07**

(21) Application number: **18801935.0**

(22) Date of filing: **14.05.2018**

(51) International Patent Classification (IPC):
**C12Q 1/6883** (2018.01)    **C12N 15/11** (2006.01)

(52) Cooperative Patent Classification (CPC):
**C12Q 1/6883; C12Q 2600/158**

(86) International application number:
**PCT/CN2018/086671**

(87) International publication number:
**WO 2018/210201 (22.11.2018 Gazette 2018/47)**

(54) **MOLECULAR MARKER, KIT AND APPLICATION FOR USE IN EARLY DIAGNOSIS AND PREDICTION OF SEPSIS AS COMPLICATION OF ACUTE KIDNEY INJURY**

MOLEKULARER MARKER, KIT UND VERWENDUNG ZUR VERWENDUNG IN DER FRÜHDIAGNOSE UND PROGNOSE VON SEPSIS ALS KOMPLIKATION EINER AKUTEN NIERENLÄSION

MARQUEUR MOLÉCULAIRE, KIT ET APPLICATION DESTINÉS À ÊTRE UTILISÉS DANS LE DIAGNOSTIC PRÉCOCE ET LA PRÉDICTION D'UNE SEPTICÉMIE EN TANT QUE COMPLICATION D'UNE LÉSION RÉNALE AIGUË

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.05.2017 CN 201710358057**

(43) Date of publication of application:
**25.09.2019 Bulletin 2019/39**

(73) Proprietor: **The Second Xiangya Hospital Of Central South University**
**Hunan 410011 (CN)**

(72) Inventors:
• **ZHANG, Dongshan**
  **Changsha**
  **Hunan 410011 (CN)**
• **QU, Siyuan**
  **Changsha**
  **Hunan 410011 (CN)**
• **LI, Huiling**
  **Changsha**
  **Hunan 410011 (CN)**
• **ZHANG, Pan**
  **Changsha**
  **Hunan 410011 (CN)**

(74) Representative: **Grünecker Patent- und Rechtsanwälte**
**PartG mbB**
**Leopoldstraße 4**
**80802 München (DE)**

(56) References cited:
**EP-A1- 2 719 771    WO-A1-2012/136548**
**CN-A- 107 541 563**

• JOHAN M LORENZEN ET AL: "CONCLUSIONS", CLINICAL CHEMISTRY, vol. 61, no. 1, 1 January 2015 (2015-01-01), pages 191-201, XP55692674, ISSN: 0009-9147, DOI: 10.1373/clinchem.2014.230359
• ANONYMOUS: "ID: URS00008B4413; Sequence, Description, Publications", NONCODE, NONCODE.ORG , 31 January 2015 (2015-01-31), pages 1-4, XP009519739, Retrieved from the Internet: URL:http://www.noncode.org/ [retrieved on 2018-03-22]
• CHUN-MEI HUANG ET AL: "Expression profiling and ontology analysis of circulating long non-coding RNAs in septic acute kidney injury patients.", CLINICAL CHEMISTRY AND LABORATORY MEDICINE 01 DEC 2016, vol. 54, no. 12, 1 December 2016 (2016-12-01), pages e395-e399, XP009520300, ISSN: 1437-4331

**(Cont. next page)**

- Anonymous: "ID: URS00008B4413; Sequence, Description, Publications", NONCODE, 31 January 2015 (2015-01-31), pages 1-4, XP009519739,
- Xu Jianru: "Reseacrh advance of early biomarkers on acute kidney injury in sepsis", Chinese Journal of Biochemical Pharmaceutics, vol. 37, no. 3, 28 March 2017 (2017-03-28) , pages 27-29, XP009515679,
- ZHANG ZH.: "Biomarkers, diagnosis and management of sepsis-induced acute ki dney injury: a narrative review", Heart, lung and Vessels, vol. 7, no. 1, 1 January 2015 (2015-01-01) , pages 64-73, XP055614988,
- Papaooannou Terzi et al: "Alphal-microglobulin as an early biomarker of sepsis-associated acute kidney injury: a prospective cohort study", Hippokratia, vol. 18, no. 3, 1 January 2014 (2014-01-01), pages 262-268, XP055614992,

Remarks:

The complete document including Reference Table(s) and the Sequence Listing(s) can be downloaded from the EPO website

## Description

## BACKGROUND

### Technical Field

The present invention relates to the technical field of molecular diagnosis, in particular to a molecular marker and a kit for early diagnosis and prediction of sepsis acute kidney injury and application thereof.

### Related Art

Sepsis is one of the common causes and complications of critically ill patients, and the mortality caused thereby is 50% or above. Meanwhile, sepsis is also a common cause of acute kidney injury in severe patients. Once sepsis patients have acute kidney injury, the mortality can reach 70%. Therefore, early diagnosis and treatment are the key of reducing the morbidity and mortality of sepsis-related AKI. At present, the diagnosis of acute kidney injury mainly depends on serum creatinine and urine volume. However, serum creatinine and urine volume are affected by various factors, so renal function changes cannot be accurately reflected in time and there is not sufficient sensitivity and specificity for AKI diagnosis. It is now known that creatinine concentration changes only when renal function loss reaches 50%, and it takes several days for creatinine to reach a steady state, so renal functions cannot be reflected in time. In addition, creatinine is affected by multiple extra-renal factors such as age, sex, race, body volume, muscle catabolism, protein intake, gastrointestinal hemorrhage and drugs. It can be seen that the increase in serum creatinine often lags behind the deterioration of renal functions and cannot accurately reflect the change of renal functions. Therefore, it is critical to find highly sensitive and specific biomarkers for early diagnosis and prognosis evaluation of AKI.

Long-chain non-coding RNA is a non-coding RNA with a length of more than 200 nucleotides. Research shows that the expression of the long-chain non-coding RNA becomes abnormal during the occurrence and development of various diseases so as to reflect the development and prognosis of diseases, and the expression of the long-chain non-coding RNA in blood is relatively stable. Therefore, the long-chain non-coding RNA has the advantages of high specificity, sensitivity, rapidness, convenience and pertinence as a molecular marker.

Document CHUN-MEI HUANG ET AL: "Expression profiling and ontology analysis of circulating long non-coding RNAs in septic acute kidney injury patients.", CLINICAL CHEMISTRY AND LABORATORY MEDICINE 01 DEC 2016, vol. 54, no. 12, discloses differentially expressed LncRNAs that are associated with septic AKI.

## SUMMARY

A first objective of the present invention is to provide a molecular marker for early detection of sepsis acute kidney injury, which is of great significance for early diagnosis and prediction of sepsis acute kidney injury.

A molecular marker TCONS_00024536 for early diagnosis and prediction of sepsis acute kidney injury, the sequence thereof being shown in SEQ ID NO: 1.

A second objective of the present invention is to provide application of the molecular marker TCONS_00024536, that is, the application of a product for detecting the expression level of TCONS_00024536 in preparing tools for early diagnosis and prediction of sepsis acute kidney injury.

The product comprises a preparation for detecting the expression level of TCONS_00024536 by RT-PCR or real-time quantitative PCR.

Primer sequences for specific amplification of TCONS_00024536 to detect the expression level of TCONS_00024536 by RT-PCR are as follows:

    F: 5'-TAGGAAGGGCTGTTGACTGG -3'
    R: 5'-CTGGGAGCTGGATTCAGAAG -3'.

A third objective of the present invention is to provide a kit for early diagnosis and prediction of sepsis acute kidney injury, which comprises a reagent for detecting the expression level of TCONS_00024536 by RT-PCR or real-time quantitative PCR.

The kit for early diagnosis and prediction of sepsis acute kidney injury comprises a pair of primers for specific amplification of TCONS_00024536 by RT-PCR, and primer sequences thereof are as follows:

    F: 5'-TAGGAAGGGCTGTTGACTGG -3'

    R: 5'-CTGGGAGCTGGATTCAGAAG -3'.

**Beneficial effects**

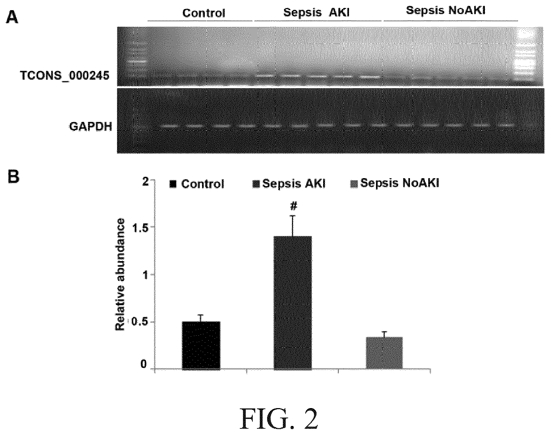

The applicant found that the expression of TCONS_00024536 was up-regulated in patients with sepsis acute kidney injury (Fig. 2), suggesting that TCONS_00024536 is a molecular marker for diagnosis and prediction of sepsis acute kidney injury. The present invention provides a strong molecular biological basis for diagnosis and prediction of sepsis acute kidney injury, and has profound clinical significance and popularization.

**BRIEF DESCRIPTION OF THE DRAWINGS**

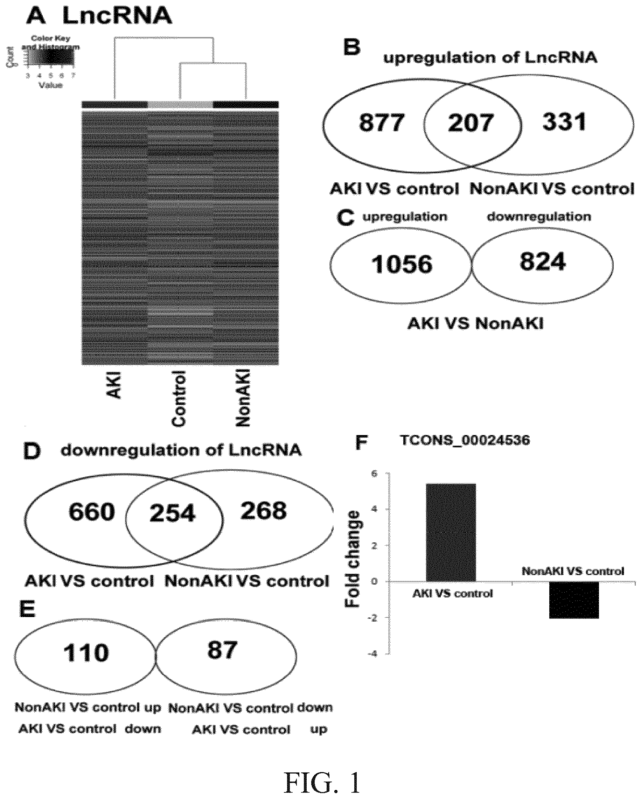

Fig. 1 shows a result of chip expression profile analysis of LncRNA.

A. heat map; B. LncRNA up-regulated in sepsis AKI compared with control group and sepsis non-AKI compared with control group; C. LncRNA up-regulated and down-regulated in sepsis AKI compared with sepsis non-AKI; D. LncRNA down-regulated in sepsis AKI compared with control group and sepsis non-AKI compared with control group; E. LncRNA up-regulated in sepsis non-AKI compared with control group while down-regulated in sepsis AKI compared with control group, and LncRNA up-regulated in sepsis non-AKI compared with control group while down-regulated in sepsis AKI compared with control group; F. TCONS_00024536 up-regulated in sepsis AKI compared with control group and down-regulated in sepsis non-AKI compared with control group.

Fig. 2 shows RT-PCR verifying the expression of TCONS_00024536 in control, sepsis AKI and sepsis non-AKI, wherein A shows TCONS_00024536 and internal reference GAPDH, and B is gray analysis. Difference is statistically significant.

**DETAILED DESCRIPTION**

The following embodiments are intended to further illustrate the present invention rather than limiting the present invention.

Embodiment 1: Screening Molecular Markers Associated with Sepsis Acute Kidney Injury

1. Sample collection

Blood samples of healthy people, sepsis non-AKI patients and sepsis AKI patients were collected.

2. Preparation and quality analysis of RNA sample

Total RNA was extracted using Trizol from Kangwei Century Company, specifically comprising the following steps:

1) 2 mL of whole blood was taken from a test tube treated with sodium citrate and put into an enzyme-free centrifuge tube;

2) plasma collection: centrifuging was performed at 3000 rpm for 10 min, and a supernatant (plasma) was carefully sucked from the top of the sample and put into another enzyme-free centrifuge tube;

3) 250 µL of plasma liquid was taken and transferred to a 1.5 ml centrifuge tube containing 750 µL of Trizol reagent, and the tube was shaken manually and violently till even mixing;

4) the homogenized sample was incubated at 15-30°C for 5 min;

5) 0.2 mL of chloroform was added to the homogenized sample and the tube was covered tightly by a tube cap, incubation was performed at 15-30°C for 2-3 min after manually shaking the tube violently for 15 sec, and centrifuging was performed at 4°C and 12,000 rpm for 15 min;

6) the mixed liquid after centrifugation was divided into a red phenol chloroform phase in the lower layer and a colorless aqueous phase in the middle layer and the upper lay, RNA was completely distributed in the aqueous phase, and 500 µL of aqueous phase was sucked and transferred to a new centrifuge tube;

7) 500 µL of isopropyl alcohol was added to the new centrifuge tube and the mixture was evenly mixed to precipitate RNA therein, incubation was performed at 15-30°C for 10 min after even mixing, and then centrifuging was performed at 4°C and 12,000 rpm for 10 min;

8) the supernatant was removed, at least 1 mL of 75% ethanol was added, the RNA precipitate was washed, and centrifuging was performed at 4°C and 7,500 rpm for 5 min after shaking;

9) the ethanol solution was removed and the RNA precipitate was dried in the air for 5-10 min;

(10) 20 μL of RNA-free enzyme water was added, blowing was performed several times with a pipette, and then the centrifuge tube containing RNA was covered and stored in a refrigerator of -80°C; and

11) RNA quality analysis: the concentration and purity of the extracted RNA were measured using NanoDrop® ND-1000.

3. High-throughput transcriptome sequencing

1) RNA-seq read localization
2) Transcription abundance assessment
3) Detection of differentially expressed genes

4. Results

The RNA-seq results are shown in Fig. 1. Plasma from 5 healthy controls, 15 sepsis non-AKI patients and 15 sepsis AKI patients were studied using an LncRNA chip in a pilot experiment. It can be known from the analysis of microarray results that:

(1) compared with healthy people, the expression of 1084 lncRNAs was up-regulated and the expression of 914 lncRNAs was down-regulated in blood samples of sepsis AKI patients;
(2) compared with healthy people, the expression of 538 lncRNAs was up-regulated and the expression of 522 lncRNAs was down-regulated in plasma of sepsis non-AKI patients; (3) compared with sepsis non-AKI, the expression of 1,056 lncRNAs was up-regulated and the expression of 824 lncRNAs was down-regulated in sepsis AKI; (4) compared with the control group, the expression of 207 lncRNAs was up-regulated and the expression of 254 lncRNAs was down-regulated in both sepsis non-AKI and sepsis AKI; (5) the expression of 110 lncRNAs was up-regulated in sepsis non-AKI compared with the control group and down-regulated in sepsis AKI compared with the control group, and the expression of 87 lncRNAs was down-regulated in sepsis non-AKI compared with the control group and up-regulated in sepsis AKI compared with the control group; and (6) TCONS_00024536 was up-regulated by 5.2 times in sepsis AKI compared with the control group and down-regulated by 2 times in sepsis non-AKI compared with the control group. The gene chip results show that LncRNA expression is changed in both sepsis AKI and non-AKI. LncRNAs up-regulated in AKI and down-regulated in non-AKI were finally screened through comparison of various groups, with the focus on TCONS_00024536 which significantly changes, suggesting that LncRNA can be used as a marker.

In order to further verify the results of the chip, the expression of TCONS_00024536 by RT-PCR was further verified.

Embodiment 2: RT-PCR Verification of Differential Expression

1. According to the detection results of high-throughput sequencing, RT-PCR verification was selected. Blood samples of healthy people, sepsis AKI patients and sepsis non-AKI patients were collected according to the sample collection method in Embodiment 1.
2. The RNA extraction procedure was the same as that in Embodiment 1.
3. Reverse transcription: a reverse transcription kit from ThermoFish was used.

| RNA | 0.1 ng-5 μg |
| --- | --- |
| Primer: Oligo (dT) 18 primer, or | 1 μL |

(continued)

| Random Hexamer primer, or gene-specific primer | 1 μL 15-20 pmol |
|---|---|
| Enzyme-free water | Add to 12 μL |
| Place on the Eppendorf PCR instrument to incubate at 65°C for 5 min, then put on ice immediately | |
| 5X Reaction Buffer | 4 μL |
| RiboLock RNase Inhibitor (20 U/μL) | 1 μL |
| 10 mM dNTP Mix | 2 μL |
| Revert Aid M-MuLV RT (200 U/μL) | 1 μL |
| Total volume | 20 μL |
| Ionize an EP tube, | |
| Place on the Eppendorf PCR instrument to incubate at 25°C for 5 min, then incubate at 42°C for 60 min, finally incubate at 70°C for 5 min to end the reaction | |

4. RT-PCR amplification

1) Primer design

According to the gene coding sequences of TCONS_00024536 and Gapdh, PCR amplification primers were designed and synthesized by Sangon Biotech in Shanghai. The specific primer sequences are as follows:

TCONS_00024536:

F: 5'-TAGGAAGGGCTGTTGACTGG -3'

R: 5'-CTGGGAGCTGGATTCAGAAG -3'

Gapdh gene:

F: 5'-CAAGGTCATCCATGACAACTTTG-3'

R: 5'-GTCCACCACCCTGTTGCTGTAG-3'

2) A PCR reaction system was prepared according to the following table (wherein the Tap MasterMix premix system was purchased from Kangwei Century Company):

| Reagent | 20 μL reaction system |
|---|---|
| 2X Tap MasterMix | 10 μL |
| Forward Primer, 10 μM | 2 μL |
| Reverse Primer, 10 μM | 2 μL |
| Template DNA | 2 μL |
| ddH$_2$O | 4 μL |

PCR reaction procedure

| Step | Temperature | Time |
|---|---|---|
| Initial denaturation | 94°C | 2 min |
| Denaturation | 94°C | 30s |
| Annealing | 55°C | 30s |
| Extension | 72°C | 30s |
| Final extension | 72°C | 2 min |

Denaturing and annealing were repeated for 35-40 cycles.

5. Agarose gel electrophoresis: 10 μL of PCR product was taken for electrophoresis detection on 2% agarose gel, agarose electrophoresis photos of RT-PCR results were subjected to gray scanning by an image analysis system, and the gray scanning value (optical density scanning value) was IA, which represented the brightness of a target band on the gel, reflecting the amount of the target band.

$$\text{Relative amount of PCR product (IA ratio)} = \text{IA of target fragment/IA of internal control}$$

$$\text{GAPDH}$$

The IA ratio of cDNA fragments in the healthy control group, the sepsis AKI group and the sepsis non-AKI group was calculated by the above formula, and the ratio was the relative amount of the RT-PCR product.

6. Results: as shown in Fig. 2, the expression of TCONS_00024536 in the blood of sepsis AKI patients was up-regulated compared with healthy people and the sepsis non-AKI group. RT-PCR results verified the discovery of the LncRNA chip, suggesting that TCONS_00024536 can be used as an early diagnostic molecular marker for sepsis AKI.

The above description of the embodiments is at least used for understanding the method of the present invention and the core idea thereof. Several improvements and modifications can be made to the present invention without departing from the principle of the present invention, and these improvements and modifications will also fall within the protection scope of the claims of the present invention.

**Claims**

**1.** A method for diagnosing and/or predicting a sepsis acute kidney injury in a patient, the method comprising:

- providing a test sample of blood obtained from said patient;
- preparing an RNA sample from said test sample;
- detecting the expression level of the LncRNA molecule TCONS_00024536 having a nucleotide sequence as set forth in SEQ ID NO: 1 in said RNA sample;
- using the detected level of expression of said LncRNA molecule TCONS_00024536 to diagnose and/or predict sepsis acute kidney injury in the patient,
wherein the detection of the expression level of the LncRNA molecule TCONS_00024536 is performed by real-time quantitative PCR, and
wherein prior to detecting the level of expression of the LncRNA molecule TCONS_00024536, said LncRNA molecule is subjected to amplification by polymerase chain reaction (PCR) using a pair of primers specific to said LncRNA molecule, wherein said pair of primers comprises a first primer consisting of a nucleotide sequence according to SEQ ID NO: 2 and a second primer consisting of a nucleotide sequence according to SEQ ID NO: 3.

**2.** A method for diagnosing and/or predicting a sepsis acute kidney injury in a patient, the method comprising:

- providing a test sample of blood obtained from said patient;
- preparing an RNA sample from said test sample;
- detecting the expression level of the LncRNA molecule TCONS_00024536 having a nucleotide sequence as set forth in SEQ ID NO: 1 in said RNA sample;

- comparing said expression level with a control expression level of said LncRNA molecule TCONS_00024536 present in an RNA sample which is prepared from a blood sample obtained from a healthy patient;
- wherein a difference in said expression level as compared to said control expression level is used for diagnosing and/or predicting sepsis acute kidney injury in the patient,

wherein a higher level of expression of said LncRNA molecule TCONS_00024536 when compared to said control expression level, correlates with the presence of a sepsis acute kidney injury,

wherein the detection of the expression level of the LncRNA molecule TCONS_00024536 is performed by real-time quantitative PCR, and

wherein prior to detecting the level of expression of the LncRNA molecule TCONS_00024536, said LncRNA molecule is subjected to amplification by polymerase chain reaction (PCR) using a pair of primers specific to said LncRNA molecule, wherein said pair of primers comprises a first primer consisting of a nucleotide sequence according to SEQ ID NO: 2 and a second primer consisting of a nucleotide sequence according to SEQ ID NO: 3.

3. A kit for diagnosis and/or prediction of sepsis acute kidney injury in a patient, comprising a reagent for detecting the expression level of the LncRNA molecule TCONS_00024536 having a nucleotide sequence as set forth in SEQ ID NO: 1, wherein said detection is performed by real-time quantitative PCR,

in which said reagent comprises a pair of primers for specific amplification of the LncRNA molecule TCONS_00024536, wherein said pair of primers comprises a first primer consisting of a nucleotide sequence according to SEQ ID NO: 2 and a second primer consisting of a nucleotide sequence as set forth in to SEQ ID NO: 3.

4. A reagent for detecting the expression level of the LncRNA molecule TCONS_00024536 having a nucleotide sequence as set forth in SEQ ID NO: 1, wherein the detection is made by real-time quantitative PCR, said reagent comprising a pair of primers for specific amplification of said LncRNA molecule, wherein said pair of primers comprises a first primer consisting of a nucleotide sequence as set forth in SEQ ID NO: 2 and a second primer consisting of a nucleotide sequence according to SEQ ID NO: 3.

5. *In vitro* use of the reagent of claim 4 in detecting the expression level of the LncRNA molecule TCONS_00024536 according to the method of claims 1 or 2, in which detecting the expression level of the LncRNA molecule is made by real-time quantitative PCR.

**Patentansprüche**

1. Verfahren zum Diagnostizieren und/oder Vorhersagen einer akuten Nierensepsis in einem Patienten, wobei das Verfahren umfasst:

- Bereitstellen einer ersten Testprobe, die von dem Patienten erhalten wird;
- Herstellen einer RNA-Probe aus der Testprobe;
- Erfassen des Exprimierungsgrades des LncRNA-Moleküls TCONS_00024536, das eine Nukleotidsequenz hat, wie sie in SEQ ID NR: 1 angegeben ist, in der RNA-Probe;
- Verwenden des erfassten Exprimierungsgrades des LncRNA-Moleküls TCONS_00024536, um eine akute Nierensepsis in dem Patienten zu diagnostizieren und/oder vorherzusagen,

wobei das Erfassen des Exprimierungsgrades des LncRNA-Moleküls TCONS_00024536 durch quantitative Echtzeit-PCR ausgeführt wird, und

wobei vor dem Erfassen des Exprimierungsgrades des LncRNA-Moleküls TCONS_00024536 das LncRNA-Molekül einer Verstärkung durch Polymerase-Kettenreaktion (PCR) unterzogen wird, wobei ein Paar aus Primer-Substanzen verwendet wird, die spezifisch für das LncRNA-Molekül sind, und wobei das Paar aus Primer-Substanzen eine erste Primer-Substanz bestehend aus einer Nukleotidsequenz gemäß der SEQ ID NR: 2 und eine zweite Primer-Substanz aufweist, die aus einer Nukleotidsequenz gemäß SEQ ID NR: 3 besteht.

2. Verfahren zum Diagnostizieren und/oder Vorhersagen einer akuten Nierensepsis in einem Patienten, wobei das Verfahren umfasst:

- Bereitstellen einer Testprobe aus Blut, das von dem Patienten erhalten wird;
- Herstellen einer RNA-Probe aus der Testprobe;
- Erfassen des Exprimierungsgrades des LncRNA-Moleküls TCONS_00024536, das eine Nukleotidsequenz hat, wie sie in SEQ ID NR: 1 angegeben ist, in der RNA-Probe;

- Vergleichen des Exprimierungsgrades mit einem Kontroll-Exprimierungsgrad des LncRNA-Moleküls TCONS_00024536, das in der RNA-Probe vorhanden ist, die aus einer von einem gesunden Patienten gewonnenen Blutprobe hergestellt wird;

- wobei ein Unterschied des Exprimierungsgrades im Vergleich zu dem Kontroll-Exprimierungsgrad zum Diagnostizieren und/oder Vorhersagen einer akuten Nierensepsis in dem Patienten verwendet wird, wobei ein höherer Exprimierungsgrad des LncRNA-Moleküls TCONS_00024536 im Vergleich zu dem Kontroll-Exprimierungsgrad mit dem Vorhandensein einer akuten Nierensepsis korreliert, wobei vor dem Erfassen des Exprimierungsgrades des LncRNA-Moleküls TCONS_00024536 das LncRNA-Molekül einer Verstärkung durch Polymerase-Kettenreaktion (PCR) unterzogen wird, wobei ein Paar von Primer-Substanzen verwendet wird, die spezifisch für das LncRNA-Molekül sind, und wobei das Paar aus Primer-Substanzen eine erste Primer-Substanz bestehend aus einer Nukleotidsequenz gemäß der SEQ ID NR: 2 und eine zweite Primer-Substanz aufweist, die aus einer Nukleotidsequenz gemäß SEQ ID NR: 3 besteht.

3. Kit zur Diagnose und/oder Vorhersage einer akuten Nierensepsis in einem Patienten, mit einem Reagens zum Erfassen des Exprimierungsgrades des LncRNA-Moleküls TCONS_00024536 mit einer Nukleotidsequenz, wie sie in SEQ ID NR: 1 angegeben ist, wobei das Erfassen durch quantitative Echtzeit-PCR erfolgt, wobei das Reagens ein Paar aus Primer-Substanzen für eine spezifische Verstärkung des LncRNA-Moleküls TCONS_00024536 aufweist, wobei das Paar aus Primer-Substanzen eine erste Primer-Substanz bestehend aus einer Nukleotidsequenz nach SEQ ID NR: 2 und einer zweiten Primer-Substanz bestehend aus einer Nukleotidsequenz, wie sie in SEQ ID NR: 3 angegeben ist, aufweist.

4. Reagens zur Erfassung des Exprimierungsgrades des LncRNA-Moleküls TCONS_00024536 mit einer Nukleotidsequenz, die in SEQ ID NR: 1 angegeben ist, wobei das Erfassen durch quantitative Echtzeit-PCR erfolgt, wobei das Reagens ein Paar aus Primer-Substanzen für spezifische Verstärkung des LncRNA-Moleküls aufweist, wobei vor dem Erfassen des Exprimierungsgrades des LncRNA-Moleküls TCONS_00024536 das LncRNA-Molekül einer Verstärkung durch Polymerase-Kettenreaktion (PCR) unterzogen wird, wobei ein Paar aus Primer-Substanzen verwendet wird, die spezifisch für das LncRNA-Molekül sind, und wobei das Paar aus Primer-Substanzen eine erste Primer-Substanz bestehend aus einer Nukleotidsequenz gemäß der SEQ ID NR: 2 und eine zweite Primer-Substanz aufweist, die aus einer Nukleotidsequenz gemäß SEQ ID NR: 3 besteht.

5. In-vitro-Verwendung eines Reagens nach Anspruch 4 beim Erfassen des Exprimierungsgrades des LncRNA-Moleküls TCONS_00024536 gemäß dem Verfahren nach Anspruch 1 oder 2, wobei Erfassen des Exprimierungsgrades des LncRNA-Moleküls durch quantitative Echtzeit-PCR erfolgt.

**Revendications**

1. Procédé de diagnostic et/ou de prédiction d'une lésion rénale aigüe septique chez un patient, le procédé comprenant :

- la fourniture d'un échantillon d'essai de sang obtenu auprès dudit patient ;
- la préparation d'un échantillon d'ARN à partir dudit échantillon d'essai ;
- la détection du niveau d'expression de la molécule d'ARNlnc TCONS_00024536 ayant une séquence nucléotidique telle que présentée dans la SEQ ID N° 1 dans ledit échantillon d'ARN ;
- l'utilisation du niveau d'expression détecté de ladite molécule d'ARNlnc TCONS_00024536 afin de diagnostiquer et/ou prédire une lésion rénale aigüe septique chez le patient, dans lequel la détection du niveau d'expression de ladite molécule d'AR-Nlnc TCONS_00024536 est effectuée par PCR quantitative en temps réel, et dans lequel avant de détecter le niveau d'expression de ladite molécule d'ARNlnc TCONS_00024536, ladite molécule d'ARNlnc est soumise à une amplification par réaction en chaîne de la polymérase (PCR) en utilisant une paire d'amorces spécifiques de ladite molécule d'ARNlnc, ladite paire d'amorces comprenant une première amorce constituée d'une séquence nucléotidique selon la SEQ ID N° 2 et une seconde amorce constituée d'une séquence nucléotidique selon la SEQ ID N° : 3.

2. Procédé de diagnostic et/ou de prédiction d'une lésion rénale aigüe septique chez un patient, le procédé comprenant :

- la fourniture d'un échantillon d'essai de sang obtenu auprès dudit patient ;
- la préparation d'un échantillon d'ARN à partir dudit échantillon d'essai ;
- la détection du niveau d'expression de la molécule d'ARNlnc TCONS_00024536 ayant une séquence nucléotidique telle que présentée dans la SEQ ID N° 1 dans ledit échantillon d'ARN ;

- la comparaison dudit niveau d'expression avec un niveau d'expression témoin de ladite molécule d'ARNlnc TCONS_00024536 présente dans un échantillon d'ARN préparé à partir d'un échantillon de sang obtenu auprès d'un patient sain ;

- dans lequel une différence dudit niveau d'expression par rapport audit niveau d'expression témoin est utilisée pour diagnostiquer et/ou prédire une lésion rénale aigüe septique chez le patient,

dans lequel un niveau plus élevé d'expression de ladite molécule d'AR-Nlnc TCONS_00024536 par rapport audit niveau d'expression témoin est corrélé avec la présence d'une lésion rénale aigüe septique,

dans lequel la détection du niveau d'expression de ladite molécule d'AR-Nlnc TCONS_00024536 est effectuée par PCR quantitative en temps réel, et

dans lequel avant de détecter le niveau d'expression de ladite molécule d'ARNlnc TCONS_00024536, ladite molécule d'ARNlnc est soumise à une amplification par réaction en chaîne de la polymérase (PCR) en utilisant une paire d'amorces spécifiques de ladite molécule d'ARNlnc, ladite paire d'amorces comprenant une première amorce constituée d'une séquence nucléotidique selon la SEQ ID N° 2 et une seconde amorce constituée d'une séquence nucléotidique selon la SEQ ID N° : 3.

3. Trousse de diagnostic et/ou de prédiction d'une lésion rénale aigüe septique chez un patient, comprenant un réactif permettant de détecter le niveau d'expression de la molécule d'ARNlnc TCONS_00024536 ayant une séquence nucléotidique telle que présentée dans la SEQ ID N° 1, la détection du niveau d'expression de ladite molécule d'ARNlnc TCONS_00024536 étant effectuée par PCR quantitative en temps réel, et

dans laquelle ledit réactif comprend une paire d'amorces destinée à l'amplification spécifique de ladite molécule d'ARNlnc TCONS_00024536, ladite paire d'amorces comprenant une première amorce constituée d'une séquence nucléotidique selon la SEQ ID N° 2 et une seconde amorce constituée d'une séquence nucléotidique selon la SEQ ID N° : 3.

4. Réactif permettant de détecter le niveau d'expression de la molécule d'ARNlnc TCONS_00024536 ayant une séquence nucléotidique telle que présentée dans la SEQ ID N° 1, la détection étant effectuée par PCR quantitative en temps réel, ledit réactif comprenant une paire d'amorces destinée à l'amplification spécifique de ladite molécule d'ARNlnc, ladite paire d'amorces comprenant une première amorce constituée d'une séquence nucléotidique selon la SEQ ID N° 2 et une seconde amorce constituée d'une séquence nucléotidique selon la SEQ ID N° : 3.

5. Utilisation *in vitro* du réactif selon la revendication 4 dans la détection du niveau d'expression de la molécule d'ARNlnc TCONS_00024536 selon le procédé des revendications 1 ou 2, la détection du niveau d'expression de la molécule d'ARNlnc étant effectuée par PCR quantitative en temps réel.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHUN-MEI HUANG et al.** Expression profiling and ontology analysis of circulating long non-coding RNAs in septic acute kidney injury patients. *CLINICAL CHEMISTRY AND LABORATORY MEDICINE,* 01 December 2016, vol. 54 (12